**Europäisches Patentamt**

**European Patent Office**

**Office européen des brevets**

(11) Veröffentlichungsnummer: **0 032 159**

**A1**

(12) **EUROPÄISCHE PATENTANMELDUNG**

(21) Anmeldenummer: **80100112.4**

(22) Anmeldetag: **10.01.80**

(51) Int. Cl.³: **A 61 B 5/02**

(43) Veröffentlichungstag der Anmeldung:
**22.07.81** Patentblatt **81/29**

(84) Benannte Vertragsstaaten:
**AT BE CH FR GB IT LU NL SE**

(71) Anmelder: **Yoo, Dae Joon, Dr. med.**
**Am Mühlenteich 33**
**D-2082 Uetersen(DE)**

(72) Erfinder: **Yoo, Dae Joon, Dr. med.**
**Am Mühlenteich 33**
**D-2082 Uetersen(DE)**

(74) Vertreter: **Hauck, Hans, Dipl.-Ing. et al,**
**Patentanwälte Dipl.-Ing.H.Hauck, Dipl.-Phys.W.Schmitz,**
**Dipl.-Ing.E.Graalfs, Dipl.-Ing.W.Wehnert,**
**Dipl.-Phys.W.Carstens Dr.-Ing.W.Döring Neuer Wall 41**
**D-2000 Hamburg 36(DE)**

(54) **Elektronisches Diagnosegerät für druckabhängige Pulsmessung und -aufzeichnung.**

(57) Die Erfindung geht aus von einem elektronischen Diagnosegerät zur druckabhängigen Pulsmessung und -aufzeichnung, bei dem drei Pulssonden (1,2,3) mit einem Verstärker (13) verbunden sind, der seinerseits mit einer motorisch (66) angetriebenen Aufzeichnungsvorrichtung (15) verbunden ist, ferner eine vierte, 19, mit einer akustischen Anzeige (22) verbundene Pulssonde einer Blutdruckmanschette zugeordnet ist, die an eine Blutdruckmeßvorrichtung (55) und eine Druckquelle (57) angeschlossen ist, wobei die Druckquelle (57) auch an eine weitere aufblasbare Manschette (40 ... 46) anschließbar ist, mittels der die drei Pulssonden angelegt werden.

EP 0 032 159 A1

Croydon Printing Company Ltd.

PATENTANWALTE
DR.-ING. H. NEGENDANK (-1973)

0032159

DIPL.-ING. H. HAUCK · DIPL.-PHYS. W. SCHMITZ · DIPL.-ING. E. GRAALFS
DIPL.-ING. W. WEHNERT · DIPL.-PHYS. W. CARSTENS · DR.-ING. W. DÖRING

HAMBURG · MÜNCHEN · DÜSSELDORF

· PATENTANWÄLTE · NEUER WALL 41 · 2000 HAMBURG 36 ·

Dr. med. Dae Joon Yoo
Am Mühlenteich 33

2082 Uetersen

SCHMITZ · GRAALFS
NEUER WALL 41 · 2000 HAMBURG 36
TELEFON + TELECOPIER (040) 36 07 55
TELEX 02 11 769 INPAT D
CABLE NEGEDAPATENT HAMBURG

HAUCK · CARSTENS
MOZARTSTRASSE 23 · 8000 MÜNCHEN 2
TELEFON + TELECOPIER (089) 53 92 86
CABLE NEGEDAPATENT MÜNCHEN

WEHNERT · DÖRING
K.-WILH.-RING 41 · 4000 DÜSSELDORF 11
TELEFON (0211) 57 50 27/28
· TELEX 08 584 889 DYNA D
CABLE NEGEDAPATENT DÜSSELDORF

ZUSTELLUNGSANSCHRIFT / PLEASE REPLY TO:       HAMBURG, 9. Januar 1980

## Elektronisches Diagnosegerät für druckabhängige Pulsmessung und -aufzeichnung

Die Erfindung geht aus von einem elektronischen Diagnosegerät zur druckabhängigen Pulsmessung und -aufzeichnung, bei dem drei Pulssonden mit einem Verstärker verbunden sind, der seinerseits mit einer motorisch angetriebenen Aufzeichnungsvorrichtung verbunden ist, ferner eine vierte, mit einer akustischen Anzeige verbundene Pulssonde einer Blutdruckmanschette zugeordnet ist, die an eine Blutdruckmeßvorrichtung und eine Druckquelle angeschlossen ist, wobei die Druckquelle auch an eine weitere aufblasbare Manschette anschließbar ist, mittels der die drei Pulssonden angelegt werden.

Ein derartiges Gerät ist bereits bekannt (US-PS 4,066,066). Es dient zur elektronischen Aufzeichnung des Pulses an drei

.../2

0032159

in der chinesischen Akupunkturliteratur angegebenen Stellen. Die erhaltenen Pulsformen werden mit in der Literatur vorhandenen Beschreibungen und Zeichnungen verglichen und in Beziehung gesetzt,um dem Arzt die Diagnosestellung zu erleichtern.

Als Pulssonden dienen zumeist piezo-elektrische Sensoren, die mit Hilfe einer Pulsmanschette an die besagten Stellen am Unterarm angelegt werden. Der Andruck wird dabei ähnlich reguliert wie in der aufblasbaren Manschette eines bekannten Blutdruckmeßgerätes. Im bekannten Fall sind Pulsmanschette und Blutdruckmanschette über eine gemeinsame Luftleitung an die Druckquelle angeschlossen. Letztere wird von einem Kompressor gebildet, der mit Hilfe eines elektrischen Motors angetrieben wird.

Abgesehen von dem verhältnismäßig hohen Aufwand zur Erzeugung eines variablen Druckes, erweist sich die gemeinsame Verbindung der Manschetten an einer Druckquelle für die Durchführung der Pulsmessung und Pulsaufzeichnung als unvorteilhaft.

Der Erfindung liegt daher die Aufgabe zugrunde, ein elektronisches Diagnosegerät zur druckabhängigen Pulsmessung und -aufzeichnung zu schaffen, welches einfach aufgebaut und bedienbar ist sowie der Diagnosestellung optimal angepaßt ist.

Diese Aufgabe wird erfindungsgemäß bei einem Diagnosegerät nach dem Oberbegriff des Anspruches 1 dadurch gelöst, daß Pulsmanschette und Blutdruckmanschette über getrennte Anschlüsse an das Diagnosegerät angeschlossen sind, eine Umschaltvorrichtung vorgesehen ist zur wahlweisen Verbindung der Manschettenanschlüsse mit der Druckquelle mit Hilfe von Betätigungsorganen und die Blutdruckmeßvorrichtung ständig mit der Druckquelle verbunden ist und eine getrennte Anzeige für systolischen und diastolischen Druck besitzt.

Die Pulsmessung bzw. Pulsaufzeichnung erfolgt unter unterschiedlichen Drücken, d. h. unterschiedliche Anpressung der Impulssonden mit Hilfe der Pulsmanschette, wobei üblicherweise eine Aufzeichnung bei einem Druck, der leicht über dem diastolischen Blutdruck liegt, einem Druck, der leicht unter dem systolischen Blutdruck liegt und bei einem mittleren Druck, der zwischen dem systolischen und diastolischen Druck liegt, erfolgt. Für die Aufzeichnung ist daher erforderlich, vorher systolischen und diastolischen Druck zu ermitteln. Dies geschieht mit Hilfe der Erfindung zunächst separat, indem die Druckquelle, welche vorzugsweise eine Gummiballpumpe ist, nur an die Blutdruckmanschette angeschlossen ist. Mit Hilfe der Anzeigevorrichtung wird dann in an sich bekannter Weise diastolischer und systolischer Druck gemessen. Anschließend erfolgt eine Umschaltung der Druckquelle an die Pulsmanschette, welche nun mit dem gewünschten Druck aufgepumpt werden kann. Da die Anzeigevorrichtung weiterhin eingeschaltet bleibt, kann der Aufpumpdruck der Pulsmanschette wirksam kontrolliert werden.

Die Aufzeichnung der Pulsformen erfolgt mit Hilfe der Aufzeichnungsvorrichtung, in der ein Papierstreifen motorisch vorgetrieben wird, auf dem dann die Pulsformen aufgetragen werden. Eine Ausgestaltung der Erfindung sieht zusätzlich vor, daß den Pulssonden zugeordnete getrennte Leuchtanzeigen für die Pulsamplituden vorgesehen sind. Die getrennten Leuchtanzeigen zeigen zwar
stets nur die Augenblickswerte an, können jedoch zur Überwachung
der Aufzeichnung verwendet werden.

Zur Überwachung des gesamten Aufzeichnungsvorgangs ist es ferner
von Vorteil, diese optisch zu kontrollieren. Dies kann gemäß einer
weiteren Ausgestaltung der Erfindung geschehen, wenn der Aufzeichnungsstreifen der Aufzeichnungsvorrichtung an der Vorderseite des
Gerätes austritt, vorzugsweise im oberen Bereich und mittels einer
Führung annähernd parallel zur Vorderseite nach unten geführt ist.
Dadurch tritt der Aufzeichnungsstreifen gut sichtbar aus dem erfindungsgemäßen Diagnosegerät heraus und gibt dem diagnosestellenden Arzt bereits einen ersten Anhalt.

Für eine umfassende Diagnose ist erforderlich, die Pulssonden an
beiden Unterarmen anzulegen. Hierfür sieht eine weitere Ausgestaltung der Erfindung vor, daß zweimal drei Anschlüsse für die
Pulssonden vorgesehen sind und eine elektrische Umschaltvorrichtung vorgesehen ist für die wahlweise Verbindung eines Anschlußsatzes an den Verstärker. Für diesen Fall ist es auch vorteilhaft,
wenn zwei Anschlüsse für die Pulsmanschette vorgesehen sind und
die Umschaltvorrichtung eine wahlweise Verbindung der Druckquelle
mit einem der Anschlüsse bewirkt. Es sind somit nur eine Pulsmanschette und ein Satz Pulssonden erforderlich, um eine Aufzeichnung
für beide Unterarme zu ermöglichen.

Ein Ausführungsbeispiel der Erfindung wird nachfolgend anhand von Zeichnungen näher beschrieben. Es zeigen:

Fig. 1   ein schematisches Blockschaltbild des Diagnosegerätes
         nach der Erfindung,

Fig. 2   die Vorderansicht des Diagnosegerätes,

Fig. 3   die Rückansicht des Gerätes nach Fig. 2,

Fig. 4   die Draufsicht auf die Innenseite einer schematisch
         dargestellten Pulsmanschette und daran befestigter
         Pulssonden und

Fig. 5   die Außenseite der Pulsmanschette nach Fig. 4
         in 180 ° gedrehter Lage.

Zwei Pulssondensätze, von denen jeder drei Pulssonden enthält,
die auf piezo-elektrischer Basis arbeiten, sind über
entsprechende Leitungen und eine elektrische Umschaltvorrichtung 12 wahlweise mit einem Verstärker 13 verbindbar. Der
Ausgang des Verstärkers 13 ist wahlweise über eine elektrische
Umschaltvorrichtung 14 mit einem Schreiber 15 oder einem Oszilloskopen 16 verbindbar. Mit Hilfe des Oszilloskopen 16 lassen
sich drei verschiedene Kurven gleichzeitig aufzeichnen, wie
durch die Ziffern 1 bis 3 deutlich gemacht. Der Schreiber
15 kann drei Kurven parallel aufzeichnen, wie durch die
Ziffern 1 bis 3 am Ausgang des Schreibers 15 kenntlich gemacht.

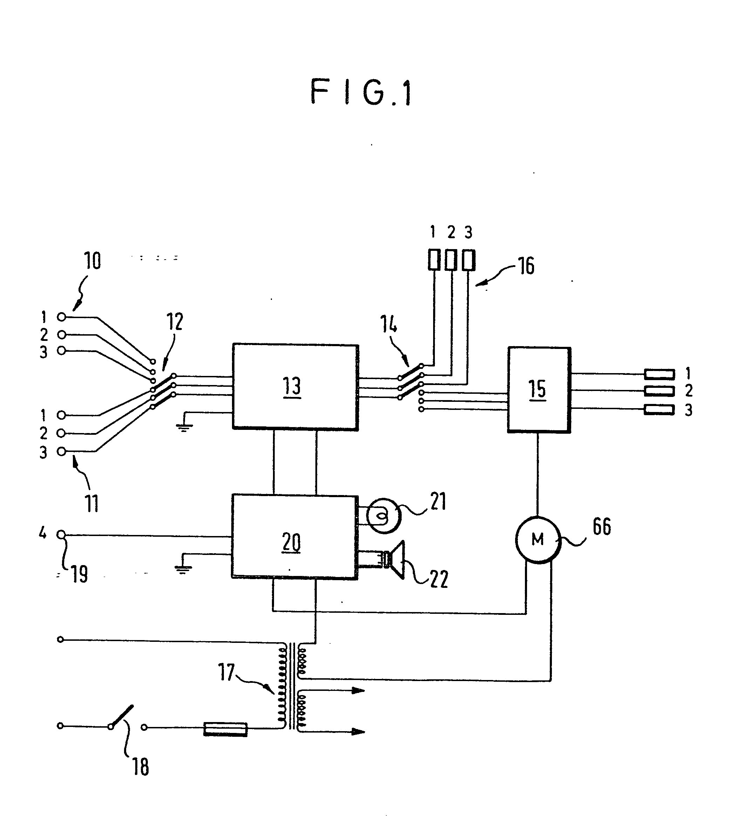

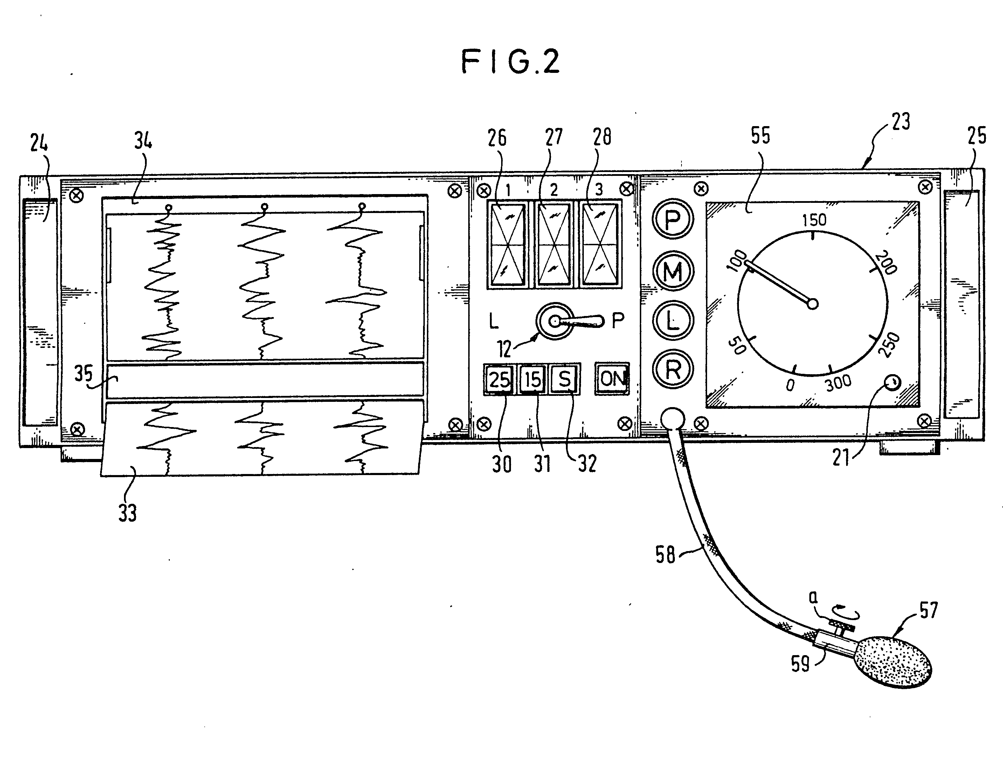

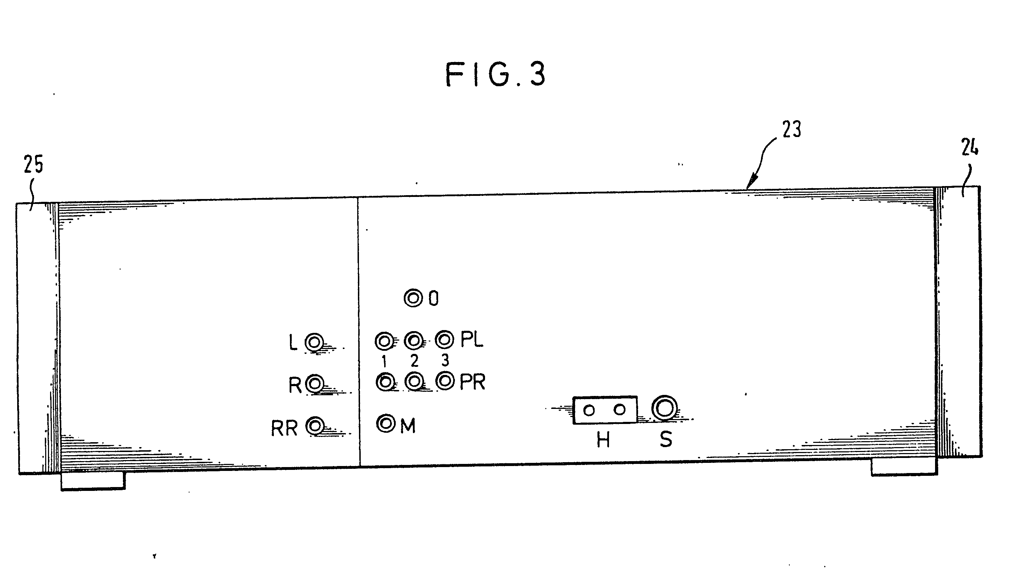

Der Schreiber 15 wird mit Hilfe eines Motors 66 angetrieben, der über einen Transformator 17 und einen Hauptschalter 18 an Netzspannung liegt. Eine vierte Pulssonde 19 liegt am Eingang eines Verstärkers 20,an dessen Ausgang eine Leuchtanzeige 21 und ein akustischer Signalgeber 22 angeschlossen sind. Die Fig. 2 und 3 zeigen ein Einschubgehäuse 23 mit seitlichen Griffen 24, 25, das die in Fig. 1 dargestellten Teile enthält. Darüber hinaus enthält es eine Blutdruckmeßvorrichtung, angedeutet durch deren Anzeige 55, drei Leuchtanzeigen 26, 27, 28 für die optische Anzeige der Pulsamplitude sowie etliche Schalter, auf die weiter unten noch einzugehen sein wird. Mit der Vorderseite des Gehäuses 23 ist außerdem eine Gummiballpumpe 57 über einen Schlauch 58 angeschlossen, die ein Ventil 59 enthält.

Die Rückseite des Gehäuses 23 nach Fig. 2 enthält zwei Anschlußsätze PL und PR für die Pulssonden 10 und 11 nach Fig. 1. Der Anschluß für das Oszilloskop 16 ist mit O bezeichnet. Der Anschluß für die Pulssonde 19 ist mit M und der Netzanschluß mit H bezeichnet. S deutet eine Sicherung an.

An den Anschluß RR wird eine nicht gezeigte Blutdruckmanschette angeschlossen, der die Pulssonde 19 zugeordnet ist. An die Anschlüsse L und R kann wahlweise eine nicht gezeigte Pulsmanschette angeschlossen werden, die zum Anlegen der Pulssonden 10 bzw. 11 dient.

Die Diagnose mit Hilfe der gezeigten Gerätes geschieht wie folgt.

Zunächst wird der Blutdruck gemessen. Hierzu wird die Blutdruckmanschette angelegt und der Sensor 19 gegen die Oberarmarterie gelegt. Durch Drücken der Taste P an der Vorderseite des Gehäuses 23 werden die Blutdruckmeßvorrichtung entlüftet und die Zeiger der Blutdruckanzeige 55 auf Null gestellt. Das Ventil 59 der Gummiballpumpe wird über seinen Knopf a geschlossen. Anschließend wird die Manschette mit Hilfe der Gummiballpumpe 27 aufgepumpt. Die Taste M zur automatischen Blutdruckmessung wird außerdem gedrückt. Die rote Leuchte 21 (siehe auch Fig. 1) an der Anzeigetafel für die Blutdruckmessung leuchtet auf bei Erreichen des systolischen Drucks bei gleichzeitigem Ticken des akustischen Signalgebers 22. Bei Erreichen des diastolischen Druckes erlöschen die Leuchte 21 und das akustische Signal. Der systolische und der diastolische Druck können nun an den zum Stillstand gekommenen Zeigern der Anzeige 55 abgelesen werden. Durch Öffnen des Ventils 59 wird der Manschettendruck abgelassen, und die Zeiger der Anzeige 55 können durch erneutes kurzes Drücken der Taste P wieder in die Nullstellung gebracht werden.

Anschließend erfolgt die Pulsaufzeichnung. Hierzu wird der ausgestreckte rechte oder linke Arm des zu Untersuchenden mit der Dorsalseite auf die ausgebreitete nicht gezeigte Gummimanschette der Pulsmanschette gelegt. Die Pulssonden 1 bis 3 werden dann an folgenden Stellen angelegt:

Pulssonde 1 an Pulstaststelle Daumen (Chon)

Pulssonde 2 an der Pulstaststelle Barriere (Kwan)

Pulssonde 3 an Pulstaststelle Fuß (Chuk)

Die Gummimanschette wird vorsichtig und locker um das Handgelenk herumgewickelt. Dann wird die äußere Stoffmanschette herumgelegt, wobei ein Verrutschen der Pulssonden 10 bzw. 11 zu vermeiden ist.

Je nach dem, ob die Pulsmanschette am linken bzw. am rechten Arm angelegt ist, wird die Taste L bzw. R gedrückt. Zuvor wurde der Kippschalter 12 (siehe auch Fig. 1) entsprechend auf L bzw. R gelegt.

Anschließend wird die Pulsmanschette gleichmäßig und langsam aufgepumpt bis zum gewünschten Manschettendruck wie folgt:

Zur Aufzeichnung des oberflächlichen Pulses:

  Diastolischer Blutdruck + 10 mm Hg

Zur Aufzeichnung des tiefen Pulses:

  Systolischer Blutdruck - 10 mm Hg

Zur Aufzeichnung des mittleren Pulses:

  Mittlerer Druck zwischen systolischem und diastolischem Druck

Vor der Aufzeichnung des Pulsverlaufes wird eine der Wahltasten 30 bzw. 31 (Fig. 2) für die Papiervorschubgeschwindigkeit gedrückt, die wahlweise 15 mm/Sek. oder 25 mm/Sek. beträgt. Schließlich wird die Starttaste 32 für den Motor 66 (Fig. 1) gedrückt.

Die Vorratsrolle für das Papier 33 ist innerhalb des Gehäuses 23 angeordnet, ebenso wie die Schreibvorrichtung. An der Vorderseite im oberen linken Bereich weist das Gehäuse 23 einen Schlitz 34 auf, über den das Papier 33 austritt. Unterhalb des Schlitzes parallel dazu ist eine bügelartige Führung 35 angeordnet, hinter der das Papier 33 entlanggeführt ist, so/daß die aufgezeigten Pulsformen gut sichtbar sind.

Durch erneutes Drücken der Taste 32 wird der Papiervorschub mit Hilfe des Motors 16 gestoppt.

Zur Kontrolle der Pulsamplitude mit oder ohne Papiervorschub dienen die Leuchtanzeigen 26 bis 28.

Wahlweise kann auch ein Oszilloskop 16 an den Anschluß o' an der Rückseite des Gehäuses 23 angeschlossen werden.

Mit Hilfe des beschriebenen Gerätes besteht die Möglichkeit, bestehende oder im Entstehen begriffene Krankheiten mit Hilfe differenzierter Pulsmessung und Pulsaufzeichnung zu diagnostizieren und den Therapieeffekt und -verlauf zu kontrollieren und zu archivieren.

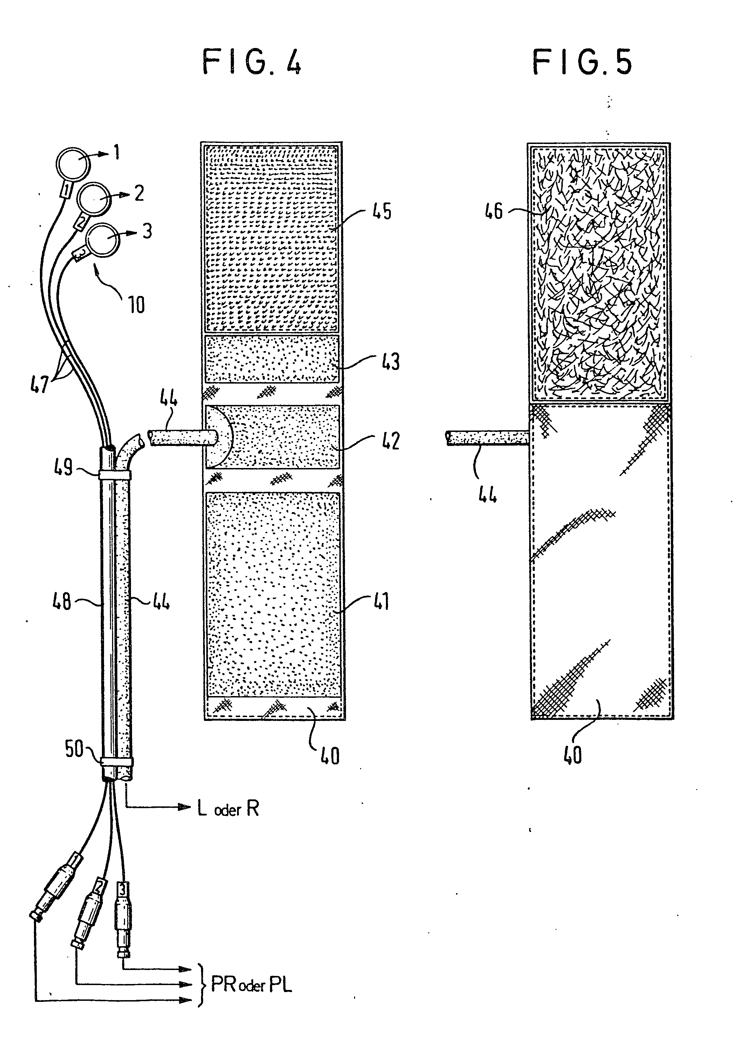

Die Pulsmanschette nach Fig. 4 und Fig. 5 besitzt eine äußere durchgehende Stofflage 40, die innen teilweise mit einer Gummischicht belegt ist, nämlich bei 41, 42 und 43, wobei die in der Mitte angeordnete Schicht 42 als aufblasbares Kissen ausgebildet ist, das mit einem Blasschlauch 44 verbunden ist. Der Blasschlauch

L oder
kann wahlweise mit Löchern/R an der Rückseite des Gehäuses
23 verbunden werden.

Die Restfläche der Innenseite ist mit einer positiven Klettenverschließschicht 45 belegt, die mit einer negativen Klettenverschlußschicht 46 auf der Außenseite der Stofflage 40 zusammenwirkt.

Die Leitungen 47 der Pulssonden 10 verlaufen gebündelt durch
einen Schutzschlauch 48, der mit Hilfe von Bändern 49, 50 am
Blasschlauch 43 befestigt ist. Die Befestigung kann auch mittels Klemmen, Blech- oder Drahtschleifen oder dergleichen erfolgen.

Das Anlegen der Pulssonden 10 an die vorgeschriebenen Punkte
wird durch die Gummischicht 41 bewirkt, die ein Verrutschen
der Sonden verhindert. Der erforderliche Andruck wird vom aufblasbaren Kissen 42 bewirkt.

P a t e n t a n s p r ü c h e:

1. Elektrisches Diagnosegerät zur druckabhängigen Pulsmessung und -aufzeichnung, bei dem drei Pulssonden mit einem Verstärker verbunden sind, der seinerseits mit einer motorgetriebenen Aufzeichnungsvorrichtung verbunden ist, ferner eine vierte mit einer akustischen Anzeige verbundene Pulssonde einer Blutdruckmanschette zugeordnet ist, die an eine Blutdruckmeßvorrichtung und eine Druckquelle angeschlossen ist, wobei die Druckquelle auch an eine weitere aufblasbare Manschette anschließbar ist, mit der drei Pulssonden angelegt werden, dadurch gekennzeichnet, daß Pulsmanschette und Blutdruckmanschette über getrennte Anschlüsse (LR; RR) an das Diagnosegerät (23) angeschlossen sind, eine Umschaltvorrichtung (M bzw. LR) vorgesehen ist zur wahlweisen Verbindung der Manschettenanschlüsse mit der Druckquelle (57) mit Hilfe von Betätigungsorganen, und die Blutdruckmeßvorrichtung (55) ständig mit der Druckquelle (57) verbunden ist und eine getrennte Anzeige für systolischen und diastolischen Druck besitzt.

2. Gerät nach Anspruch 1, dadurch gekennzeichnet, daß den Pulssonden (10 bzw. 11) zugeordnete getrennte Leuchtanzeigen (26, 27, 28) für die Pulsamplitude vorgesehen sind.

3. Gerät nach Anspruch 1 oder 2, dadurch gekennzeichnet, daß der Aufzeichnungsstreifen (33) der Aufzeichnungsvorrichtung an der Vorderseite des Gerätes (23) austritt, vorzugsweise im oberen Bereich und mittels einer Führung (35) annähernd parallel zur Vorderseite nach unten geführt ist.

4. Gerät nach einem der Ansprüche 1 bis 3, dadurch gekennzeichnet, daß zweimal drei Anschlüsse für die Pulssonden (10 bzw. 11) vorgesehen sind und eine elektrische Umschaltvorrichtung (12) vorgesehen ist für die wahlweise Verbindung eines Anschlußsatzes an den Verstärker (13).

5. Gerät nach Anspruch 4, dadurch gekennzeichnet, daß zwei Anschlüsse (LR) für die Pulsmanschette vorgesehen sind und die Umschaltvorrichtung eine wahlweise Verbindung der Druckquelle (57) mit den Anschlüssen (LR) bewirkt.

6. Gerät nach einem der Ansprüche 1 bis 5, gekennzeichnet durch eine Steuervorrichtung für den Motor (66) für den Streifenvorschub, die eine stufenweise Änderung der Vorschubgeschwindigkeit ermöglicht.

7. Gerät nach einem der Ansprüche 1 bis 6, gekennzeichnet durch einen am Gehäuse (23) vorgesehenen, mit dem Ausgang des Verstärkers (13) verbundenen Anschluß (O) für ein Oszilloskop.

8. Gerät nach einem der Ansprüche 1 bis 7, dadurch gekennzeichnet, daß die Pulsmanschette aus einer Außenlage, vorzugsweise aus Stoff, und einer inneren Gummilage besteht, und nur ein begrenzter Abschnitt der Gummilage als aufblasbares Kissen ausgebildet ist.

9. Gerät nach einem der Ansprüche 1 bis 8, dadurch gekennzeichnet, daß die Leitungen für die Pulssonden von einer schlauchartigen Hülle umgeben sind und die Hülle am Blasschlauch für
die Pulsmanschette befestigt ist.

# FIG.1

# FIG.2

# FIG.3

L ◎
R ◎
RR ◎

◎ 0

◎ ◎ ◎ PL
1 2 3
◎ ◎ ◎ PR

◎ M

H    S

25    23    24

-3-

0032159

-4-

FIG.4          FIG.5

0032159

Nummer der Anmeldung
EP 80 10 0112

Europäisches
Patentamt

**EUROPÄISCHER RECHERCHENBERICHT**

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |

<table>
<tr><td></td><td>US - A - 3 744 490 (H.S. FERNAN-DEZ)<br><br>* Zusammenfassung; Spalte 2, Zeile 10 bis Spalte 3, Zeile 48; Abbildung 1 *<br><br>--</td><td>1,2</td></tr>
<tr><td></td><td>US - A - 4 005 701 (S. AISENBERG et al./WHITTACKER CORP.)<br><br>* Zusammenfassung; Spalte 3, Zeilen 3-22; Spalte 5, Zeilen 5-46; Spalte 6, Zeile 59 bis Spalte 7, Zeile 55; Abbildungen 3,6 *<br><br>--</td><td>1,2</td></tr>
<tr><td></td><td>FR - A - 726 777 (J.R. CARRE)<br><br>* Seite 2, Zeile 72 bis Seite 3, Zeile 25; Seite 5, Zeilen 16-82; Abbildungen 1-3 *<br><br>--</td><td>1,2</td></tr>
<tr><td></td><td>FR - A - 2 374 012 (BAYER AG)<br><br>* Seite 2, Zeile 10 bis Seite 4, Zeile 6; Abbildungen 1-5 *<br><br>--</td><td>1,5,8</td></tr>
<tr><td></td><td>DE - A - 2 732 286 (FILAC CORP.)<br><br>* Seite 6, Zeile 17 bis Seite 9, Zeile 17; Abbildungen 1-3 *<br><br>--</td><td>1,5,8</td></tr>
<tr><td></td><td>FR - A - 1 233 617 (G.E. CASAL)<br><br>* Seite 1, rechte Spalte, Zeilen 3-25; Seite 2, linke Spalte, Zeile 5 bis Seite 3, linke Spalte, Zeile 3; Seite 3,<br><br>./.</td><td>1,5</td></tr>
</table>

**KLASSIFIKATION DER ANMELDUNG (Int. Cl.³)**

A 61 B 5/02

**RECHERCHIERTE SACHGEBIETE (Int. Cl.³)**

A 61 B 5/02

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 11-09-1980 | RIEB |

Europäisches
Patentamt

**EUROPÄISCHER RECHERCHENBERICHT**

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| | rechte Spalte, Zeilen 46-53; Abbildung 1 * | | |
| | -- | | |
| | DE - C - 824 823 (BOSCH & SPEIDEL) | 1,5 | |
| | * Seite 2, Zeile 30 bis Seite 3 Zeile 23; Abbildungen 1-4 * | | |
| | -- | | |
| | US - A - 3 154 358 (F.A. BLASCH/ R.L. GANNON) | 1,3 | RECHERCHIERTE SACHGEBIETE (Int. Cl.³) |
| | * Spalte 1, Zeilen 22-29; Spalte 2, Zeilen 4-7; Spalte 3, Zeilen 10-31; Spalte 4, Zeilen 43-48; Abbildungen 1-3 * | | |
| | -- | | |
| | FR - A - 904 385 (SOCIETE POUR L'EXPLOITATION DES BREVETS ET PROCEDES C. ROGUE) | 1,3 | |
| | * Seite 1, Zeile 33 bis Seite 2, Zeile 100; Abbildungen 1,2 * | | |
| | -- | | |
| | FR - A - 1 096 592 (G. PIGEON) | 1,6 | |
| | * Seite 3, linke Spalte, Zeile 30 bis rechte Spalte, Zeile 56; Abbildung 5 * | | |
| | -- | | |
| | US - A - 3 230 950 (M.A. BUFFING-TON/ THE BISHOP AND BABCOCK CORP.) | 1,7 | |
| | * Spalte 5, Zeilen 5-31; Spalte 9, Zeile 73 bis Spalte 10, Zeile 74; Abbildung 1 * | | |
| | -- ./. | | |

0032159

Europäisches
Patentamt

EUROPÄISCHER RECHERCHENBERICHT

Nummer der Anmeldung

EP 80 10.0112
-3-

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | CH - A - 562 025 (SEIWA ME LABORATORIES INC.) <br><br> * Spalte 2, Zeilen 48-57; Spalte 3, Zeile 33 bis Spalte 4, Zeile 13; Abbildungen 4-11 * <br><br> -- | 2,8,9 | |
| | US - A - 4 027 662 (A.S.J. LEE/ MILSTEIN MEDICAL RESEARCH FOUNDATION) <br><br> * Zusammenfassung; Spalte 4, Zeile 61 bis Spalte 6, Zeile 52; Spalte 12, Zeile 51 bis Spalte 13, Zeile 14; Abbildungen 1,7 * <br><br> -- | 1,2 | RECHERCHIERTE SACHGEBIETE (Int. Cl.³) |
| DA | US - A - 4 066 066 (HEE SOO PAEK) <br><br> * Zusammenfassung; Spalte 1, Zeilen 1-36; Spalte 4, Zeilen 30-61; Abbildungen 6,7 * <br><br> -- | 1 | |
| T | DE - B - 2 845 889 (DAE JOON YOO) <br><br> * Das ganze Dokument * <br><br> ---- | 1-9 | |